# EUROPEAN PATENT APPLICATION

(11) **EP 4 840 951 A1**
(43) Date of publication of application: **18.11.2026**
(21) Application number: 25738505.4
(22) Date of filing: 09.01.2025
(51) Int. Cl.: C12N 9/24, C12P 21/08, C12P 21/00, A61K 47/68

(54) **NOVEL ENDO-BETA-N-ACETYLGLUCOSAMINIDASES AND USE THEREOF**

(30) Priority: 10.01.2024 CN 202410035281; 03.01.2025 CN 202510013226
(71) Applicant: Shanghai Qilu Pharmaceutical Research and Development Centre Ltd., Shanghai 201203 (CN)
(72) Inventor: HUANG, Guoyin, Shanghai 201203 (CN); QIN, Ken, Shanghai 201203 (CN); AI, Xianwei, Shanghai 201203 (CN); FANG, Yan, Shanghai 201203 (CN); YANG, Yang, Shanghai 201203 (CN); TAO, Weikang, Shanghai 201203 (CN)
(74) Representative: Bandpay & Greuter
(86) International application number: PCT/CN2025/071512
(87) International publication number: WO 2025/148976

(57) **Abstract**

Provided are two novel endo-beta-N-acetylglucosaminidases, as well as a method for using the two with a disaccharide linker to prepare a non-natural sugar-engineered antibody, or a fixed-point quantitative antibody drug conjugate based on glycosyl modification, and a use.

## Description

The present application claims priority to Chinese Patent Application No. CN2024100352818, filed with the China National Intellectual Property Administration on January 10, 2024, entitled "NOVEL ENDO-BETA-N-ACETYLGLUCOSAMINIDASES AND USE THEREOF"; and Chinese Patent Application No. CN2025100132263, filed with the China National Intellectual Property Administration on January 03, 2025, entitled "NOVEL ENDO-BETA-N-ACETYLGLUCOSAMINIDASES AND USE THEREOF", the entire contents of which are incorporated herein by reference.

### TECHNICAL FIELD

The present disclosure relates to the field of pharmaceutical chemistry and biotechnology pharmaceuticals, and in particular to two novel beta-N-acetylglucosaminidases and new use thereof.

### BACKGROUND

Antibodies are a type of glycoprotein belonging to the immunoglobulin superfamily. At present, antibody-based drugs are widely used in many fields of disease treatment. Natural IgG antibodies comprise two light chains and two heavy chains, which are interconnected by disulfide bonds. Antibodies contains a conserved Fc region in which there is a naturally occurring and conserved N-glycosylation site (N297) at position 297. Generally, antibody-based drugs for therapy are obtained by recombinant expression. Monoclonal antibodies produced by recombinant expression in animal-derived cells as host (e.g., CHO cells), generally has a biantennary complex-type sugar chain modification. The glycosylation of antibodies can significantly affect their effector activities, including antibody-dependent cytotoxicity and complement-dependent cytotoxicity.

In addition to the naturally occurring biological functions, a variety of techniques for producing antibody-based conjugated drugs have been derived using this conserved modification site. Antibody-based conjugated drugs are a class of biotechnology drugs that use antibodies as targeting carriers, carrying an effector payload, and thereby achieving the enrichment of the effector payload in the targeted region. Loadable effector payloads include cytotoxins, radioisotopes, oligonucleotides, immunomodulators, polypeptides or protein fragments, and the like.

Antibody-Drug Conjugates (ADCs) are a class of biotechnological drugs consisting of antibodies, cytotoxins, and linkers. ADCs combines the targeting ability of the antibody with the cytotoxic killing capability, and controls the release of the cytotoxin through the linker to achieve targeted killing of tumor cells. Currently, commercially available ADCs primarily rely on naturally occurring lysine (such as Trastuzumab emtansine) or cysteine (such as Enfortumab vedotin) in the antibody. These ADCs, produced using the natural amino acid random coupling method, suffer from poor homogeneity, unstable coupling sites, leading to poor drug safety and narrow therapeutic windows. Currently, various methods are available for preparing site-specific ADCs, including exogenous cysteine insertion techniques, non-natural amino acid insertion techniques, enzymatic coupling techniques, and glycosyl site-specific conjugation techniques, among others.

Endo-beta-N-acetylglucosaminidase (ENGase) is a class of endoglycosidases with hydrolytic activity, which can hydrolyze the sugar chains attached to the antibody *in vitro*. On this basis, Laixi Wang et al., used ENGase mutants (e.g., Endo S D233Q, Endo S2 D184M) to transfer a natural or bioorthogonal functional group- bearing biantennary oxazoline substrate to the N-sugar terminus, thereby achieving *in vitro* sugar engineering of the antibody. Subsequently, the introduction of bioorthogonal functional groups allows for the preparation of antibody-based conjugated drugs. In addition, Huang Wei's team and Laixi Wang's team reported methods for preparing sugar-engineered antibodies and ADCs using ENGase, with disaccharide linkers as substrates, respectively.

The ENGase family contains a number of members, among which Endo S and Endo S2 possess hydrolytic activity and disaccharide linker transfer activity, but their broader substrate specificity has yet to be discussed. Endo Si exhibits significant hydrolytic activity, and its mutants demonstrate the transfer activity of a biantennary oxazoline substrate. However, there are no reports indicating whether Endo Si possesses disaccharide linker transfer activity. Therefore, the substrate specificity of disaccharide linkers for members of the ENGase family remains to be explored, and there is an ongoing urgent demand for the development of more efficient ENGase for industrial production.

### SUMMARY OF THE INVENTION

The present disclosure identifies and tests two endo-beta-N-acetylglucosaminidases with broader substrate specificity and better transfer efficiency, based on the development of optimized endo-beta-N-acetylglucosaminidases. Through efficient enzymatic reactions, the site-specific introduction of disaccharide linkers can be achieved, thereby realizing the sugar engineering of the antibody molecule. The bioorthogonal functional group introduced by the disaccharide linker enables the efficient preparation of the antibody-based conjugated drugs, with ease of operation and suitability for industrial production. The resulting antibody-based conjugated drugs exhibit good druggability and can be used for the treatment of tumors, inflammation, infectious diseases, or other immune diseases.

The present disclosure discovers a novel endo-beta-N-acetylglucosaminidase from *Streptococcus equi* subsp. zooepidemicus Sz105, named Endo Se2, which has the amino acid sequence set forth in SEQ ID NO: 1, with positions 1-36 being the signal peptide and positions 37-1011 being the full-length sequence of the mature enzyme. Surprisingly, this enzyme exhibits exceptional sugar chain hydrolysis and/or sugar chain transfer activity.

Furthermore, the present disclosure discovers another endo-beta-N-acetylglucosaminidase, Endo Si from *Streptococcus iniae*, with the amino acid sequence set forth in SEQ ID NO: 2, with positions 1-33 being the signal peptide and positions 34-928 being the full-length sequence of the mature enzyme. Surprisingly, this enzyme also exhibits exceptional sugar chain hydrolysis and/or sugar chain transfer activity, and exhibits significant disaccharide linker transfer activity.

Based on the discovery of the two enzymes described above, the present disclosure provides a method for sugar chain remodeling of a polypeptide or protein, comprising the following steps:
a) introducing Endo Se2 or Endo Si;
b) introducing a polypeptide or protein comprising at least one N-acetylglucosamine or core fucosylated N-acetylglucosamine;
c) providing a disaccharide linker or disaccharide conjugate; and
d) using Endo Se2 or Endo Si to transfer the disaccharide linker or disaccharide conjugate to the polypeptide or protein described in step (b), to provide a new sugar chain-modified polypeptide or protein.

In some embodiments, the polypeptide or protein is an antibody or a protein comprising the Fc region of the antibody.

In some embodiments, the polypeptide or protein comprising at least one N-acetylglucosamine or core fucosylated N-acetylglucosamine is obtained by hydrolysis of an N-glycan-containing polypeptide or protein using an endoglycosidase with endo-beta-N-acetylglucosaminidase activity, or obtained by recombinant expression, or obtained by chemical synthesis.

In some embodiments, the N-glycan is a natural or non-natural complex, high-mannose, hybrid N-glycan.

In some embodiments, the endoglycosidases with endo-beta-N-acetylglucosaminidase activity include Endo Se2, Endo Si, Endo S, Endo S2, Endo F3, and mutants thereof.

In some embodiments, the N-glycan-containing polypeptide or protein can achieve sugar chain remodeling in one step by introducing Endo Se2 or Endo Si, without the need for purification.

In some embodiments, the N-glycan-containing polypeptide or protein can achieve sugar chain remodeling in one step by simultaneously introducing Endo Se2 or Endo Si with another endoglycosidase or multiple endoglycosidases, without the need for purification.

In some embodiments, the disaccharide linker comprises at least one oxazolinylated or thiazolinylated monosaccharide, or a monosaccharide modified/engineered with a bioorthogonal functional group.

In some embodiments, the disaccharide linker is selected from the following structures:

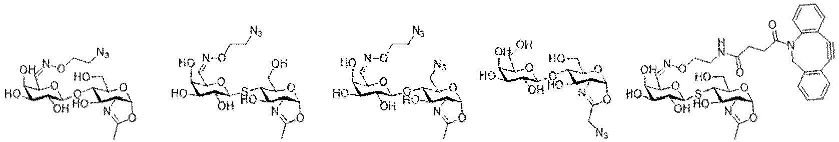

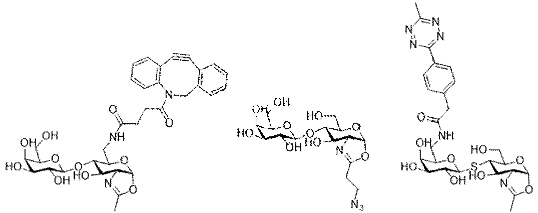

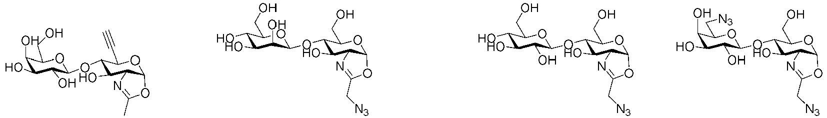

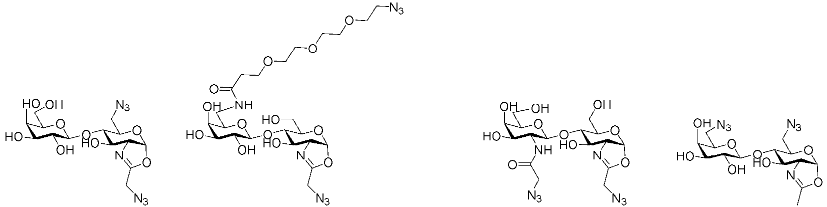

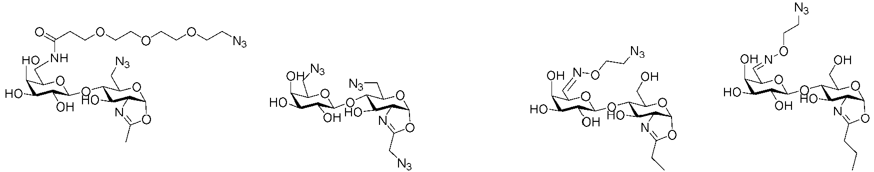

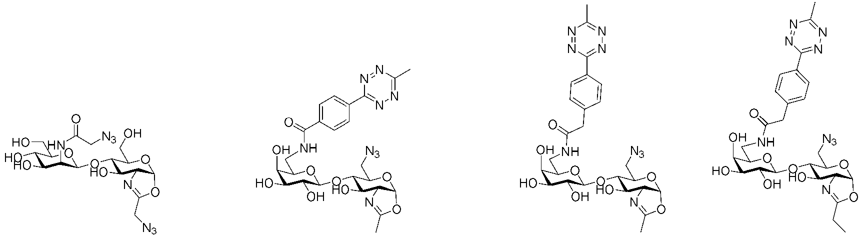

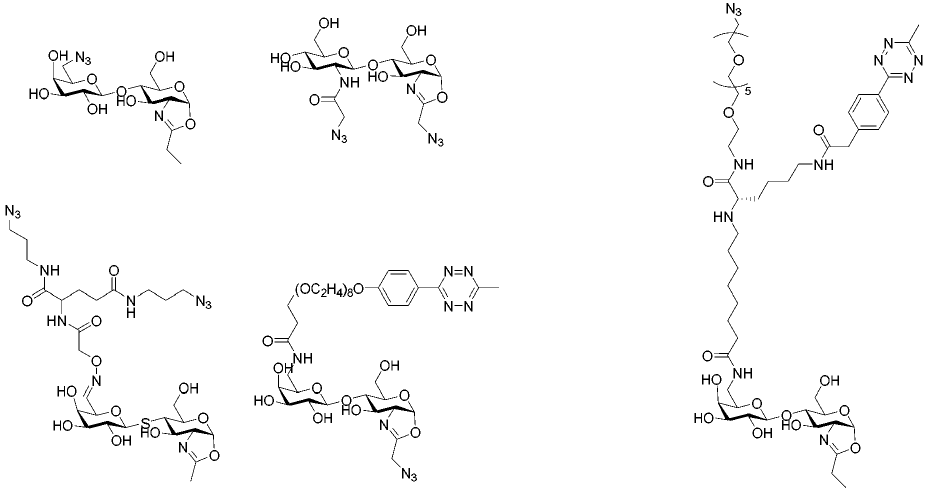

In some embodiments, in addition to comprising one oxazolinylated or thiazolinylated monosaccharide, the disaccharide conjugate comprises a toxin, a small molecule drug, a near-infrared or fluorescent probe, a polypeptide, RNA and related drugs, a radioisotope label, a contrast agent, a nuclear magnetic resonance imaging agent, and the like; and optionally, it comprises or does not comprise a cleavable linker, a non-cleavable linker, or a combination thereof. The small molecule drug is preferably selected from the group consisting of maytansine, DM-1, DM-4, MMAE, MMAF, Auristatin 0101, SN-38, Dxd, exatecan, duocarmycin, amanitin, PBD compounds, VP-16, camptothecin, paclitaxel, docetaxel, anthracyclines, and derivatives of the foregoing compounds; or the small molecule drug is a radiotherapeutic drug.

In some embodiments, the disaccharide conjugate is selected from the following structure:

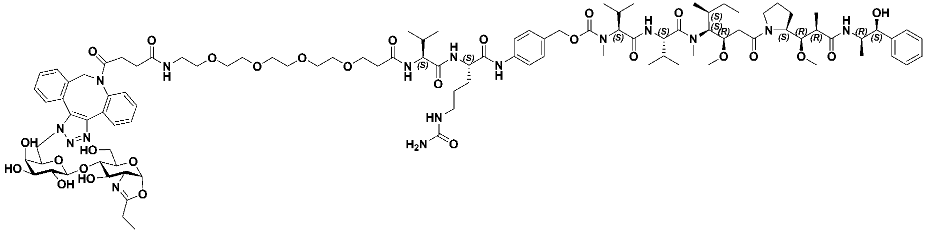

The present disclosure also provides the use of Endo Se2 or Endo Si for transferring disaccharide linkers or disaccharide conjugates in glycosyl modification.

The present disclosure enables the preparation of the homogenized sugar chain-modified antibody via the aforementioned glycoengineering modification methods. Endo Se2 or Endo Si exhibit superior transfer efficiency and broader substrate specificity compared with the prior art. The discovery and application of Endo Se2 and Endo Si provide superior tool enzymes for the structural development of disaccharide linkers, and also provide a better option for the industrial production of disaccharide linker-based sugar-engineered antibodies and antibody-based conjugated drugs derived therefrom.

### BRIEF DESCRIPTION OF THE DRAWINGS

Figure 1 shows the transglycosylation activity assays of different endoglycosidases for compound G0.
Figure 2 shows the transglycosylation activity assays of different endoglycosidases for compound G1.
Figure 3 shows the transglycosylation activity assays of different endoglycosidases for compound G2.
Figure 4 shows the efficacy of low-dose site-specific glycoconjugated ADC in NCI-N87 xenograft model.
Figure 5 shows the efficacy of high-dose site-specific glycoconjugated ADC in NCI-N87 xenograft model.

### SPECIFIC EMBODIMENTS

All publications, patents, and patent applications referred to in the specification are incorporated herein by reference to the same extent as if each individual publication, patent, or patent application was specifically and individually incorporated herein by reference.

Before the present disclosure is described in detail below, it should be understood that the present disclosure is not limited to the particular methodology, protocols, and reagents described herein, as these may vary. It should also be understood that the terminology used herein is only intended to describe specific embodiments rather than to limit the scope of the present disclosure. Unless otherwise defined, any technical and scientific term used herein has the same meaning as commonly understood by those of ordinary skill in the art to which the present disclosure belongs.

Certain embodiments disclosed herein encompass numerical ranges, and certain aspects of the present disclosure may be described by means of ranges. Unless otherwise indicated, it should be understood that the numerical ranges or the description by means of ranges are merely for the purposes of brevity and convenience, and should not be construed as a strict definition of the scope of the present disclosure. Accordingly, the description by means of ranges should be considered to have specifically disclosed all the possible subranges as well as all the possible specific numerical points within that range, as if those subranges and numerical points were explicitly written out herein. The foregoing principle applies equally regardless of the breadth of the numerical range. Where a range is employed, the range includes the endpoints thereof.

In the present disclosure, the term "antibody" includes, for example, polyclonal antibodies, monoclonal antibodies, chimeric antibodies, humanized antibodies and primatized antibodies, human antibodies, multispecific antibodies, bispecific antibodies, monovalent antibodies, multivalent antibodies, and the like, provided they exhibit the desired biological activity. The term "immunoglobulin" is used interchangeably with "antibody" herein. As used herein, the term "monoclonal antibody" refers to an antibody obtained from a substantially homogeneous population of antibodies.

The term "antibody fragment" comprises at least a portion of an intact antibody. As used herein, the "fragment" of an antibody molecule includes an "antigen-binding fragment" of an antibody, and the term "antigen-binding fragment" refers to a polypeptide fragment of an immunoglobulin or antibody specifically binding to or reacting with a selected antigen or an epitope thereof, or a fusion protein product further derived from such a fragment, e.g., a single-chain variable fragment, an extracellular binding region in a chimeric antigen receptor, and the like. Exemplary antibody fragments or antigen-binding fragments thereof include, but are not limited to: variable light chain fragments (VL), variable heavy chain fragments (VH), Fab fragments, F(ab')₂ fragments, Fd fragments, Fv fragments, single-domain antibodies, linear antibodies, single-chain variable fragment (scFv), bispecific antibodies or multispecific antibodies formed by antibody fragments, and the like.

The term "multispecific antibody" refers to a new antibody construct formed by functional attachment (e.g., chemical coupling, gene fusion, non-covalent binding, or other methods) of an antibody or antibody fragment to one or more other binding molecules (including antibodies, antibody fragments, or other molecules with binding capacity), thereby enabling binding to more than two different sites and/or targets. Thus, a "bispecific antibody" (alternatively referred to as a "bispecific antigen-binding molecule" or "BsAb") specifically refers to an antibody construct that is specific for two different antigens and/or epitopes. Generally, bispecific antibodies or multispecific antibodies comprise at least two different antigen (or epitope) binding domains.

The term "sugar" refers to a polysaccharide or an oxidized or unoxidized carbohydrate-containing molecule including, but not limited to, monosaccharides, disaccharides, trisaccharides, oligosaccharides, or polysaccharides. Sugar is also used herein to refer to the carbohydrate portion of sugar conjugates, such as glycoproteins, glycolipids, glycopeptides, glycoproteomics, peptidoglycans, lipopolysaccharides or proteoglycans. The term "sugar chain" is used interchangeably with "sugar" herein.

As used in the present disclosure, a "sugar chain linker", as an activated donor molecule for glycosyl conjugation, may be a synthetic oxazoline- or thiazoline-containing sugar, such as an oligosaccharide with an activated reducing terminus, preferably an oligosaccharide molecule structured with an oxazoline; it may also be a natural N-glycan oxazoline. Sugar chain linkers may also be chemically modified, such as by introducing functional groups through azidation, alkynylation, aldehydation, sulfhydrylation, and the like. The term "disaccharide linker" is a sugar chain linker comprising at least two monosaccharide units, preferably two monosaccharide units linked by a glycosidic or thioether bond. The term "disaccharide conjugate" is a conjugate based on a disaccharide linker and further includes payloads such as cytotoxins, and optionally also includes a linker between the disaccharide linker and the payloads.

Core fucosylated and non-fucosylated glycoproteins are important molecular classes that play a key role in many biological events such as tumor metastasis, cell adhesion, pathogen infection, and immune responses. Natural and recombinant fucosylated and non-fucosylated glycoproteins are generally produced as mixtures of glycoforms that differ only in the structure of the side chain oligosaccharides.

The term "antibody-based conjugated drug" in the present disclosure refers generally to any conjugate formed by covalently linking a polypeptide/protein targeting specific cells to a payload. The polypeptide/protein targeting specific cells may be an antibody or an antigen-binding fragment thereof, such as a mAb, a BsAb, or a MsAb, and the like; The payload may be cytotoxins, small molecule drugs, near-infrared or fluorescent probes, polypeptides, RNA and related drugs, radioisotope labels, contrast agents, nuclear magnetic resonance imaging agents, and the like; The resulting covalent conjugates can be used for therapeutic and diagnostic purposes. The term "antibody-drug conjugate" as used herein refers to any conjugate formed by covalently linking a polypeptide/protein targeting specific cells to a cytotoxin.

The term "endo-beta-N-acetylglucosaminidase" in the present disclosure refers to a class of endoglycosidase-active enzymes (EC 3.2.1.96) produced by a series of organisms, typically belonging to the glycoside hydrolase family 18 or 85. For some endo-beta-N-acetylglucosaminidases known in the art, such as Endo S, Endo S2, Endo F3, Endo A, Endo D, Endo Si, and the like, see those described in WO2022/050300. These enzymes may also concurrently possess disaccharide linker transfer activity. For enzymes known in the art with such activity, such as Endo S, Endo S2, Endo F3, and mutants thereof, see those described in WO2022/226420. The term "ENGase" is used interchangeably with "endo-beta-N-acetylglucosaminidase" herein.

A "wild-type antibody", as used in the present disclosure, typically refers to a naturally occurring or recombinantly expressed antibody possessing an N-glycosylation site (N-glycan). For example, all antibodies having an N297 site in the Fc fragment fall within the scope of "wild-type antibody", and the Fc fragment is derived from IgG1, IgG2, IgG3 or IgG4.

The term "deglycosylated antibody" in the present disclosure refers to an antibody containing one N-acetylglucosamine or core fucosylated N-acetylglucosamine, which is formed from a wild-type antibody under the action of a glycoside hydrolase; or refers to an antibody containing one N-acetylglucosamine or core fucosylated N-acetylglucosamine produced by direct recombinant expression in particular cells or prepared by chemical synthesis.

The term "sugar-engineered antibody" or "glycoengineered antibody" in the present disclosure broadly refers to an engineered antibody that achieves the homogenization of sugar chain modification *in vitro* using sugar chain engineering techniques. The sugar-engineered antibodies or glycoengineered antibodies in the present disclosure are obtained by transferring a natural or non-natural saccharide chain linker to a deglycosylated antibody, catalyzed by endo-beta-N-acetylglucosaminidase. The sugar chain modification of the sugar-engineered antibody or glycoengineered antibody may be natural or non-natural, and may carry bioorthogonal functional groups.

The antibodies used in the Examples of the present disclosure are wild-type antibodies comprising an Fc fragment whose sequence is, for example, as set forth in SEQ ID NO: 5, with pertuzumab as a typical example of the antibodies.

In the present disclosure, the fillers, columns, and instruments used for antibody, sugar chain modified antibody, ADC purification, property determination include: 5 mL cOmplete His-Tag Purification Column (Roche), HiLoad^{™} 26/600 Superdex^{™} 200 prep grade column (Cytive), AmMag^{™} Protein A Magnetic Beads (GenScript), SDA030 protein purification system (Sepure), Acquity I-Class/RDa (Waters) liquid chromatograph mass spectrometer, Arc Premier high performance liquid chromatograph, ACCQUITY UPLC BEH PROTEIN C4 (Waters, 1.7 µm, 2.1 mm×50 mm) column, TSKgel G3000SWXL (7.8 mm×30 cm, 5 µm) SEC column, TSKgel Butyl-NPR (4.6 mm×10 cm, 2.5 µm) HIC column.

In the present disclosure, the linker-payloads DBCO-GGFG-Dxd and TCO-PEG₄-GGFG-Dxd are purchased from Shanghai Tekanbio Pharm-Tech Co., Ltd.; the linker-payloads DBCO-PEG₄-GGFG-Dxd, DBCO-PEG₄-VC-PAB-MMAE, and N₃-PEG₄-VC-PAB-MMAE are purchased from MedChemExpress LLC. In this disclosure, Trastuzumab is purchased from Sanyou Biopharmaceuticals (Shanghai) Co., Ltd. In the present disclosure, sugar chain linkers were synthesized upon commission from Wuhan GLYCOGENE Pharmaceutical Co., Ltd. Other compounds and reagent, unless otherwise specified, were purchased from Sinopharm Chemical Reagent Co., Ltd.

### General Preparation Examples

### General Procedure I: Preparation of Sugar-Engineered Antibodies

The wild-type antibody, the prepared disaccharide linker, and the endoglycosidase (Endo Si or Endo Se2) were mixed to achieve concentrations of 10 mg/mL, 2 mM (30-fold equivalents of the antibody concentration), and 0.4 mg/mL, respectively. The pH of the reaction system was adjusted to 7.4, and the mixture was incubated at 25°C for 12 h. The desired non-natural sugar-engineered antibody was then obtained through protein A purification.

### General Procedure II: Site-Specific ADC Preparation Based on Azido Modified Sugar-Engineered Antibody

The prepared azide-modified sugar-engineered antibody and cycloalkyne (e.g., DBCO) linker-payload were mixed to achieve concentrations of 5 mg/mL and 0.33 mM, respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

### General Procedure III: Site-Specific ADC Preparation Based on Cycloalkynyl-Modified Sugar-Engineered Antibody

The prepared cycloalkynyl-modified sugar-engineered antibody and the azide (N₃) linker-payload were mixed to achieve concentrations of 5 mg/mL and 0.33 mM, respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

### General Procedure IV: Site-Specific ADC Preparation Based on Tetrazine-Modified Sugar-Engineered Antibody

The prepared tetrazine-modified sugar-engineered antibody and trans-cyclooctene (TCO) linker-payload were mixed to achieve concentrations of 5 mg/mL and 0.33 mM, respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

### General Procedure V: One-Step Preparation of Site-Specific Glycoconjugated ADC

The prepared payloaded disaccharide linker (i.e., disaccharide conjugate), the wild-type antibody, and the endoglycosidase (Endo Si or Endo Se2) were mixed to achieve concentrations of 0.4 mM, 5 mg/mL and 0.4 mg/mL, respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

### General Procedure VI: Dual-Payload Site-Specific ADC Preparation Based on Azide- and Tetrazine-Modified Sugar-Engineered Antibodies

The prepared azide- and tetrazine-modified sugar-engineered antibodies, cycloalkyne (e.g., DBCO) linker-payload, and TCO linker-payload were mixed to achieve concentrations of 5 mg/mL, 0.33 mM, and 0.33 mM, respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

### General Procedure VII: Site-Specific ADC Preparation Based on Linear Alkynyl-Modified Sugar-Engineered Antibody

Copper sulfate, tris (3-hydroxypropyltriazolylmethyl) amine (THPTA), and sodium ascorbate were pre-mixed to achieve concentrations of 20 mM, 100 mM, and 150 mM, respectively, and were used as catalysts. The resulting linear alkynyl-modified sugar-engineered antibody, azide linker-payload, and catalysts were mixed to achieve concentrations of 5 mg/mL, 0.5 mM, 0.67 mM (copper sulfate), 3.33 mM (THPTA), and 5 mM (sodium ascorbate), respectively. The pH of the reaction system was adjusted to 7.4, and the mixture was incubated at 25°C overnight. After LC-MS confirmed the conversion to the product, the desired site-specific glycoconjugated ADC was obtained by protein A purification or ultrafiltration.

The specific procedures for preparing sugar-engineered antibodies and site-specific glycoconjugated ADCs using the methods of preparation of the present disclosure are described below through specific Examples.

### Example 1 Recombinant Expression and Purification of Enzymes

The coding genes for the following four enzymes were cloned into the pET22b vector (GenScript). The four enzymes are Endo Se2 from *Streptococcus equi* subsp. zooepidemicus Sz105, with the amino acid sequence of 37-1011 of SEQ ID NO: 1; Endo Si from *Streptococcus iniae*, with the amino acid sequence of 34-928 of SEQ ID NO: 2; Endo S2 from *Streptococcus pyogenes* NZ131 (serotype M49), with the amino acid sequence of 38-843 of SEQ ID NO: 3, and Endo S from *Streptococcus pyogenes*, with the amino acid sequence of which is 37-995 of SEQ ID NO: 4.

The plasmids containing the genes of interest were transformed into *E. coli* BL21 (DE3) and plated on 2×YT solid plates containing 100 µg/mL ampicillin, followed by incubated at 37°C overnight. A single colony was picked and inoculated into 4 mL of 2×YT liquid medium containing 100 µg/mL ampicillin, then cultured overnight. 4 mL of bacterial solutions were pipetted into 1 L of 2×YT broth medium containing 100 µg/mL ampicillin and cultured at 37°C until OD₆₀₀ reached 0.8-1.0. Then, 0.4 mM of isopropyl β-D-1- thiogalactopyranoside (IPTG) was added to the culture, followed by incubation at 20°C to induce protein overexpression. After 16 hours, the bacterial cells were harvested by centrifugation. The cell pellets were lysed using B-PER^{™} Bacterial Protein Extraction Reagent (Thermo) following the manufacturer's instructions. The recombinant proteins of Endo S, Endo S2, Endo Si and Endo Se2 were purified using cOmplete His-Tag Purification Column (Roche), SDA030 Protein Purification System (Sepure). The solution was concentrated using Amicon centrifugal filter (30 kDa, Millipore) and further purified by size exclusion chromatography using HiLoad^{™} 26/600 Superdex^{™} 200 prep grade column (Cytiva). Fractions containing Endo S, Endo S2, Endo Si, and Endo Se2 fusion proteins were concentrated using Amicon centrifugal filters (30 kDa, Millipore) and stored in storage buffer (20 mM PB, pH 7.5). Protein purity was analyzed by sodium dodecyl sulfate-polyacrylamide gel electrophoresis (SDS-PAGE) and gel imager (Gel Doc EZ Imager, Bio-RAD), Image Lab scanning software, and protein concentrations were quantified using a spectrophotometer (Nano-300).

### Example 2 Transfer Activity Studies of Endo Se2 and Endo Si on Various Disaccharide Linkers

The wild-type antibody containing the Fc fragment of SEQ ID NO: 5 was dissolved in a buffer consisting of 20 mM phosphate, 150 mM NaCl, and pH 7.4, to achieve a final antibody concentration of 10 mg/mL, hydrolyzed by adding wild-type Endo S2 at a final concentration of 0.4 mg/mL, incubated overnight at 37°C, and purified using Protein A magnetic beads to yield a deglycosylated antibody containing an N-acetylglucosamine or core fucosylated N-acetylglucosamine.

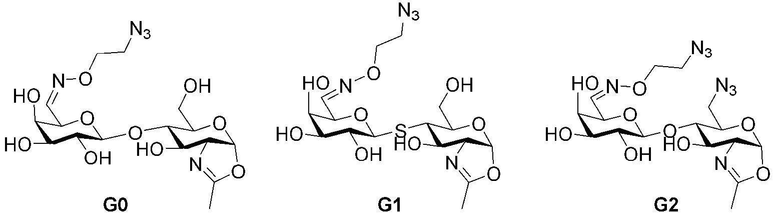

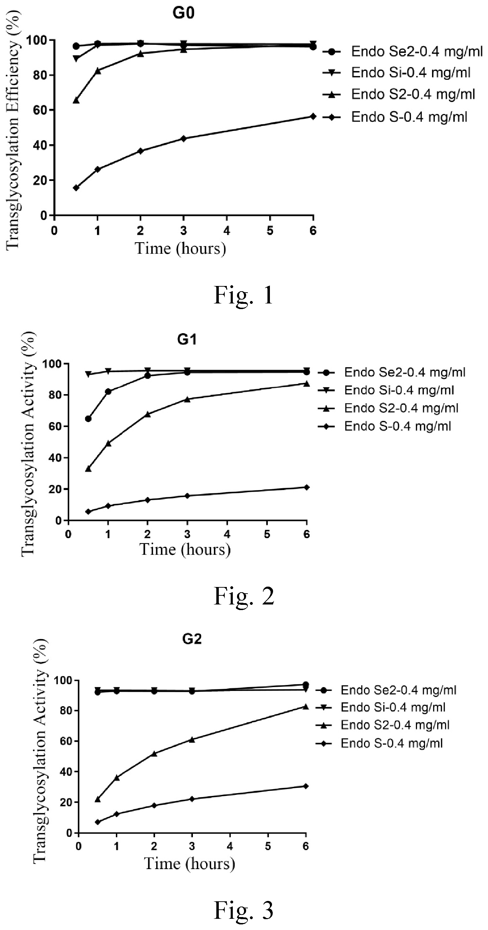

The prepared deglycosylated antibody, disaccharide linkers (i.e., compounds G0-G2), and endoglycosidase (i.e., Endo S or Endo S2 or Endo Si or Endo Se2) were mixed to achieve concentrations of 10 mg/mL, 1.67 mM (25 equivalents of the antibody concentration), and 0.4 mg/mL, respectively. The pH of the reaction system was adjusted to 7.4, and the reaction temperature was set to 25°C. 4 µL of samples were taken at 0.5, 1, 2, 3, and 6 h. The reaction was stopped by adding 36 µL of PBS containing 0.1% formic acid and analyzed by reduced LC-MS. The results are shown in Figures 1-3 and Table 1.

For compound G0, at 1 h, the transglycosylation efficiencies of Endo Se2 and Endo Si were comparable and significantly superior to those of Endo S2 and Endo S; and at 6 h, the transglycosylation efficiencies of Endo Se2, Endo Si, and Endo S2 were comparable, and significantly superior to Endo S (Figure 1, Table 1).

For compound G1, at 1 h, the transglycosylation efficiency of Endo Si was superior to Endo Se2 and significantly superior to that of Endo S2. The transfer efficiency of Endo S for compound G1 was relatively weak; at 6 h, the transglycosylation efficiencies of Endo Se2 and Endo Si were comparable and superior to that of Endo S2 (Figure 2, Table 1).

For compound G2, at 1 h, the transglycosylation efficiencies of Endo Si and Endo Se2 were comparable and significantly superior to those of Endo S2. The transfer efficiency of Endo S for compound G2 was relatively weak; at 6 h, the transglycosylation efficiencies of Endo Se2 and Endo Si were comparable and superior to those of Endo S2 (Figure 3, Table 1).

**Table 1 Transglycosylation activities of different endoglycosidases**

| Disaccharide linker | Endoglycosidase | Transglycosylation efficiency (%) | | | | |
|---|---|---|---|---|---|---|
| | | 0.5 h | 1 h | 2h | 3 h | 6h |
| G0 | Endo Se2 | 96.6 | 97.9 | 98.0 | 97.2 | 96.2 |
| | Endo Si | 89.3 | 97.1 | 97.8 | 97.8 | 97.6 |
| | Endo S2 | 65.7 | 82.7 | 92.4 | 94.8 | 97.3 |
| | Endo S | 15.7 | 26.2 | 36.6 | 43.7 | 56.4 |
| G1 | Endo Se2 | 64.8 | 82.1 | 92.4 | 94.5 | 94.7 |
| | Endo Si | 93.2 | 95.2 | 95.5 | 95.5 | 95.5 |
| | Endo S2 | 33.1 | 49.2 | 67.7 | 77.3 | 87.5 |
| | Endo S | 5.6 | 9.3 | 13.0 | 15.7 | 21.1 |
| G2 | Endo Se2 | 92.2 | 92.9 | 92.8 | 92.7 | 97.2 |
| | Endo Si | 93.4 | 93.4 | 93.3 | 93.1 | 93.8 |
| | Endo S2 | 22.2 | 36.2 | 51.9 | 61.0 | 82.7 |
| | Endo S | 7.0 | 12.3 | 17.9 | 22.1 | 30.5 |

### Examples 3 Study of the Substrate Specificity of Endoglycosidases such as Endo Se2 and Endo Si for Various Disaccharide Linkers

The wild-type Pertuzumab, disaccharide linkers, and wild-type endoglycosidase (i.e., Endo S or Endo S2 or Endo Si or Endo Se2) were mixed to achieve concentrations of 10 mg/mL, 1.67 mM (25-fold equivalents of antibody concentration), and 0.4 mg/mL, respectively. The pH of the reaction system was adjusted to 7.4, and the reaction temperature was set to 25°C. After 6 hours, 4 µL of sample was taken, and the reaction was stopped by adding 36 µL of PBS containing 0.1% formic acid and analyzed by LC-MS.

The structures of the various disaccharide linkers are as follows:

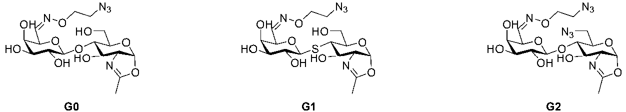

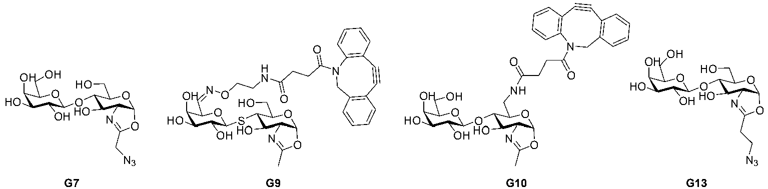

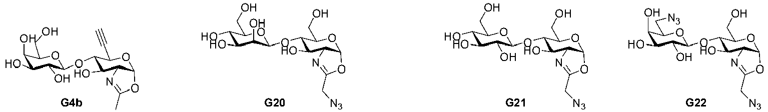

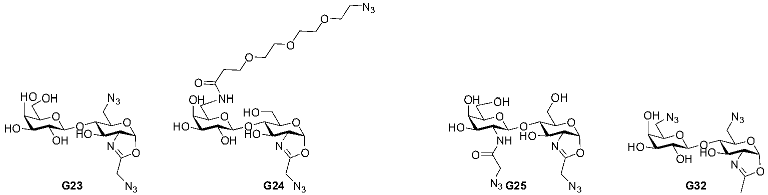

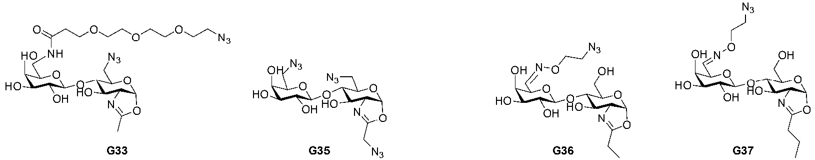

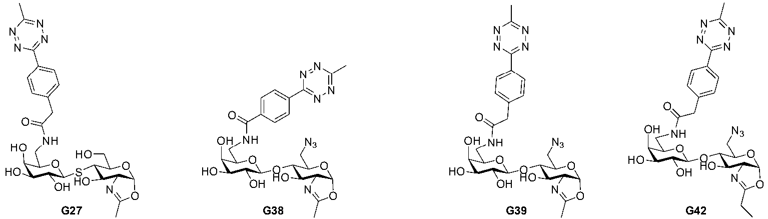

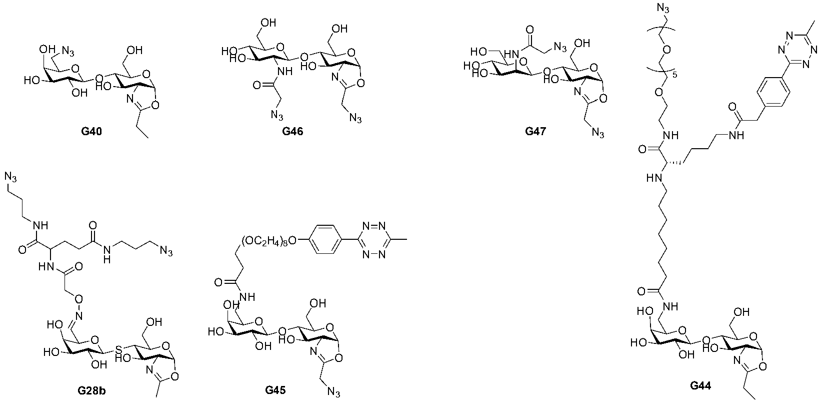

**Table 2 Transglycosylation activities of different endoglycosidase**

| Transglycosyla tion efficiency (%) | Enzyme | | | |
|---|---|---|---|---|
| | Endo Se2 | Endo Si | Endo S2 | Endo S |
| G0 | >95 | >95 | >95 | 56.4 |
| G1 | 94.7 | >95 | 87.5 | 21.1 |
| G2 | >95 | 93.8 | 82.7 | 30.5 |
| G4b | 16.1 | 38.4 | 13.9 | 17.8 |
| G7 | 76.3 | 90.6 | 36.3 | 13 |
| G9 | 36.3 | >95 | 4.7 | 7.6 |
| G10 | 13.1 | 10.1 | 8.1 | 17.4 |
| G13 | 75.3 | 51.8 | 6.9 | 0 |
| G20 | 89 | 77.7 | 89.8 | 56.7 |
| G21 | >95 | >95 | 72.6 | 29.4 |
| G22 | >95 | 68.2 | 7.7 | 0 |
| G23 | 56.7 | 93.7 | 6.0 | 2.6 |
| G24 | 88.4 | 60.8 | 29.9 | 11.7 |
| G25 | 22.5 | 21.2 | 23.6 | 15.9 |
| G27 | 34.4 | >95 | 10.2 | 5.8 |
| G28b | 94.9 | >95 | 55 | 13.6 |
| G32 | >95 | >95 | 25 | 10.8 |
| G33 | >95 | >95 | 35.3 | 24.1 |
| G35 | 80.1 | >95 | 7.8 | 6.6 |
| G36 | 80.1 | >95 | >95 | 81.4 |
| G37 | 94.2 | 51.3 | 11.7 | 11.0 |
| G38 | 17.7 | 60.7 | 6.7 | 5.1 |
| G39 | 34.1 | >95 | 23.5 | 20.9 |
| G40 | >95 | >95 | 79.9 | 33.2 |
| G42 | 59.4 | >95 | 13.1 | 9.7 |
| G44 | >95 | >95 | 52.9 | 11.7 |
| G45 | 53.1 | 15.1 | 6.9 | 0 |
| G46 | 14.9 | 21.1 | 14.0 | 14.8 |

As can be seen in Table 2, Endo Se2, Endo Si, Endo S2, and Endo S exhibit different substrate specificity and transglycosylation efficiency for different disaccharide linker compounds. Endo Se2 and Endo Si exhibit a broader substrate specificity compared to Endo S2 and Endo S. The optimal endoglycosidase for each substrate is typically Endo Se2 or Endo Si.

### Example 4 Preparation of Sugar-Engineered Antibodies Based on Wild-Type Antibodies

### Preparation Example 1: Preparation of Sugar-Engineered Antibody Ab-G1

The non-natural sugar-engineered antibody Ab-G1 was prepared from compound G1 and the wild-type antibody Pertuzumab according to General Procedure I. After deconvolution, the measured value of the HRMS of the sugar-engineered antibody Ab-G1 heavy chain was 49893.48 Da.

### Preparation Example 2: Preparation of Sugar-Engineered Antibody Ab-G2

The non-natural sugar-engineered antibody Ab-G2 was prepared from compound G2 and the wild-type antibody Pertuzumab according to General Procedure I. After deconvolution, the measured value of the HRMS of the sugar-engineered antibody Ab-G2 heavy chain was 49902.46 Da.

### Preparation Example 3: Preparation of Sugar-Engineered Antibody Ab-G4b

The non-natural sugar-engineered antibody Ab-G4b was prepared from compound G4b and the wild-type antibody Pertuzumab according to General Procedure I. After deconvolution, the measured value of the HRMS of the sugar-engineered antibody Ab-G4b heavy chain was 49789.06 Da.

### Preparation Example 4: Preparation of Sugar-EngineeredAntibody Ab-G7

The non-natural sugar engineered antibody Ab-G7 was prepared from compound G7 and the wild-type antibody Pertuzumab according to General Procedure I. After deconvolution, the measured value of the HRMS of the sugar-engineered antibody Ab-G7 heavy chain was 49810.12 Da.

### Preparation Example 5: Preparation of Sugar-Engineered Antibody Ab-G9

The non-natural sugar-engineered antibody Ab-G9 was prepared from compound G9 and the wild-type antibody Pertuzumab according to General Procedure I. After deconvolution, the measured value of the HRMS of the sugar-engineered antibody Ab-G9 heavy chain was 50154.80 Da.

### Preparation Example 6: Preparation of Sugar-Engineered Antibody Ab-G10

The non-natural sugar-engineered antibody Ab-G10 was prepared from compound G10 and the wild-type antibody Pertuzumab according to General Procedure I. After deconvolution, the measured value of the HRMS of the sugar-engineered antibody Ab-G10 heavy chain was 50081.15 Da.

### Preparation Example 7: Preparation of Sugar-Engineered Antibody Ab-G13

The non-natural sugar-engineered antibody Ab-G13 was prepared from compound G13 and the wild-type antibody Pertuzumab according to General Procedure I. After deconvolution, the measured value of the HRMS of the sugar-engineered antibody Ab-G13 heavy chain was 49807.70 Da.

### Preparation Example 8: Preparation of Sugar-EngineeredAntibody Ab-G20

The non-natural sugar engineered antibody Ab-G20 was prepared from compound G20 and the wild-type antibody Pertuzumab according to General Procedure I. After deconvolution, the measured value of the HRMS of the sugar-engineered antibody Ab-G20 heavy chain was 49836.03 Da.

### Preparation Example 9: Preparation of Sugar-Engineered Antibody Ab-G21

The non-natural sugar-engineered antibody Ab-G21 was prepared from compound G21 and the wild-type antibody Pertuzumab according to General Procedure I. After deconvolution, the measured value of the HRMS of the sugar-engineered antibody Ab-G21 heavy chain was 49835.47 Da.

### Preparation Example 10: Preparation of Sugar-Engineered Antibody Ab-G22

The non-natural sugar-engineered antibody Ab-G22 was prepared from compound G22 and the wild-type antibody Pertuzumab according to General Procedure I. After deconvolution, the measured value of the HRMS of the sugar-engineered antibody Ab-G22 heavy chain was 49835.38 Da.

### Preparation Example 11: Preparation of Sugar-Engineered Antibody Ab-G23

The non-natural sugar-engineered antibody Ab-G23 was prepared from compound G23 and the wild-type antibody Pertuzumab according to General Procedure I. After deconvolution, the measured value of the HRMS of the sugar-engineered antibody Ab-G23 heavy chain was 49835.18 Da.

### Preparation Example 12: Preparation of Sugar-Engineered Antibody Ab-G24

The non-natural sugar-engineered antibody Ab-G24 was prepared from compound G24 and the wild-type antibody Pertuzumab according to General Procedure I. After deconvolution, the measured value of the HRMS of the sugar-engineered antibody Ab-G24 heavy chain was 50064.00 Da.

### Preparation Example 13: Preparation of Sugar-EngineeredAntibody Ab-G25

The non-natural sugar-engineered antibody Ab-G25 was prepared from compound G25 and the wild-type antibody Pertuzumab according to General Procedure I. After deconvolution, the measured value of the HRMS of the sugar-engineered antibody Ab-G25 heavy chain was 49893.33 Da.

### Preparation Example 14: Preparation of Sugar-EngineeredAntibody Ab-G27

The non-natural sugar-engineered antibody Ab-G27 was prepared from compound G27 and the wild-type antibody Pertuzumab according to General Procedure I. After deconvolution, the measured value of the HRMS of the sugar-engineered antibody Ab-G27 heavy chain was 50024.69 Da.

### Preparation Example 15: Preparation of Sugar-EngineeredAntibody Ab-G28b

The non-natural sugar-engineered antibody Ab-G28b was prepared from compound G28b and the wild-type antibody Pertuzumab according to General Procedure I. After deconvolution, the measured value of the HRMS of the sugar-engineered antibody Ab-G28b heavy chain was 50175.80 Da.

### Preparation Example 16: Preparation of Sugar-Engineered Antibody Ab-G32

The non-natural sugar-engineered antibody Ab-G32 was prepared from compound G32 and the wild-type antibody Pertuzumab according to General Procedure I. After deconvolution, the measured value of the HRMS of the sugar-engineered antibody Ab-G32 heavy chain was 49844.75 Da.

### Preparation Example 17: Preparation of Sugar-Engineered Antibody Ab-G33

The non-natural sugar-engineered antibody Ab-G33 was prepared from compound G33 and the wild-type antibody Pertuzumab according to General Procedure I. After deconvolution, the measured value of the HRMS of the sugar-engineered antibody Ab-G33 heavy chain was 50047.05 Da.

### Preparation Example 18: Preparation of Sugar-EngineeredAntibody Ab-G35

The non-natural sugar-engineered antibody Ab-G35 was prepared from compound G35 and the wild-type antibody Pertuzumab according to General Procedure I. After deconvolution, the measured value of the HRMS of the sugar-engineered antibody Ab-G35 heavy chain was 49886.09 Da.

### Preparation Example 19: Preparation of Sugar-Engineered Antibody Ab-G36

The non-natural sugar-engineered antibody Ab-G36 was prepared from compound G36 and the wild-type antibody Pertuzumab according to General Procedure I. After deconvolution, the measured value of the HRMS of the sugar-engineered antibody Ab-G36 heavy chain was 49891.14 Da.

### Preparation Example 20: Preparation of Sugar-Engineered Antibody Ab-G37

The non-natural sugar-engineered antibody Ab-G37 was prepared from compound G37 and the wild-type antibody Pertuzumab according to General Procedure I. After deconvolution, the measured value of the HRMS of the sugar-engineered antibody Ab-G37 heavy chain was 49904.60 Da.

### Preparation Example 21: Preparation of Sugar-EngineeredAntibody Ab-G38

The non-natural sugar-engineered antibody Ab-G38 was prepared from compound G38 and the wild-type antibody Pertuzumab according to General Procedure I. After deconvolution, the measured value of the HRMS of the sugar-engineered antibody Ab-G38 heavy chain was 50018.20 Da.

### Preparation Example 22: Preparation of Sugar-Engineered Antibody Ab-G39

The non-natural sugar-engineered antibody Ab-G39 was prepared from compound G39 and the wild-type antibody Pertuzumab according to General Procedure I. After deconvolution, the measured value of the HRMS of the sugar-engineered antibody Ab-G39 heavy chain was 50032.24 Da.

### Preparation Example 23: Preparation of Sugar-Engineered Antibody Ab-G40

The non-natural sugar-engineered antibody Ab-G40 was prepared from compound G40 and the wild-type antibody Pertuzumab according to General Procedure I. After deconvolution, the measured value of the HRMS of the sugar-engineered antibody Ab-G40 heavy chain was 49833.19 Da.

### Preparation Example 24: Preparation of Sugar-EngineeredAntibody Ab-G42

The non-natural sugar-engineered antibody Ab-G42 was prepared from compound G42 and the wild-type antibody Pertuzumab according to General Procedure I. After deconvolution, the measured value of the HRMS of the sugar-engineered antibody Ab-G42 heavy chain was 50046.26 Da.

### Preparation Example 25: Preparation of Sugar-EngineeredAntibody Ab-G44

The non-natural sugar-engineered antibody Ab-G44 was prepared from compound G44 and the wild-type antibody Pertuzumab according to General Procedure I. After deconvolution, the measured value of the HRMS of the sugar-engineered antibody Ab-G44 heavy chain was 50624.49 Da.

### Preparation Example 26: Preparation of Sugar-Engineered Antibody Ab-G45

The non-natural sugar-engineered antibody Ab-G45 was prepared from compound G45 and the wild-type antibody Pertuzumab according to General Procedure I. After deconvolution, the measured value of the HRMS of the sugar-engineered antibody Ab-G45 heavy chain was 50405.15 Da.

### Preparation Example 27: Preparation of Sugar-Engineered Antibody Ab-G46

The non-natural sugar-engineered antibody Ab-G46 was prepared from compound G46 and the wild-type antibody Pertuzumab according to General Procedure I. After deconvolution, the measured value of the HRMS of the sugar-engineered antibody Ab-G46 heavy chain was 49865.77 Da.

### Preparation Example 28: Preparation of Sugar-EngineeredAntibody Ab-G47

The non-natural sugar-engineered antibody Ab-G47 was prepared from compound G47 and the wild-type antibody Pertuzumab according to General Procedure I. After deconvolution, the measured value of the HRMS of the sugar-engineered antibody Ab-G47 heavy chain was 49866.18 Da.

### Example 5 Preparation of Site-Specific Glycoconjugated Antibody-Drug Conjugates Based on Wild-Type Antibodies

### Preparation Example 29: Preparation of Site-Specific Glycoconjugated ADC Ab-G1-DXd

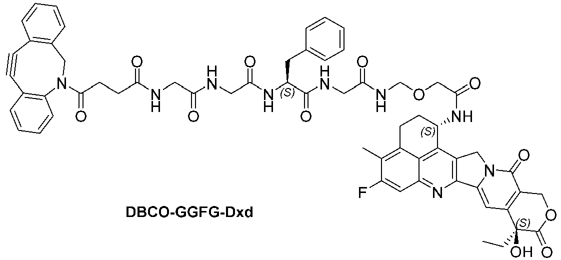

Ab-G1-DXd was prepared from compound DBCO-GGFG-Dxd and non-natural sugar-engineered antibody Ab-G1 according to General Procedure II. After deconvolution, the measured value of the HRMS of the heavy chain was 51021.77 Da.

### Preparation Example 30: Preparation of Site-Specific Glycoconjugated ADC Ab-G2-DXd

Ab-G2-DXd was prepared from compound DBCO-GGFG-Dxd and non-natural sugar-engineered antibody Ab-G2 according to General Procedure II. After deconvolution, the measured value of the HRMS of the heavy chain was 52158.93 Da.

### Preparation Example 31: Preparation of Site-Specific Glycoconjugated ADC Ab-G4b-MMAE

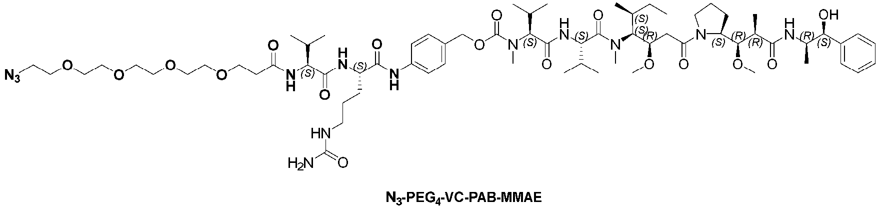

Ab-G4b-MMAE was prepared from compound N₃-PEG₄-VC-PAB-MMAE and non-natural sugar-engineered antibody Ab-G4b according to General Procedure VII. After deconvolution, the measured value of the HRMS of the heavy chain was 51185.38 Da.

### Preparation Example 32: Preparation of Site-Specific Glycoconjugated ADC Ab-G7-DXd

Ab-G7-DXd was prepared from compound DBCO-GGFG-Dxd and non-natural sugar-engineered antibody Ab-G7 according to General Procedure II. After deconvolution, the measured value of the HRMS of the heavy chain was 50964.74 Da.

### Preparation Example 33: Preparation of Site-Specific Glycoconjugated ADC Ab-G9-MMAE

Ab-G9-MMAE was prepared from compound N₃-PEG₄-VC-PAB-MMAE and non-natural sugar-engineered antibody Ab-G9 according to General Procedure III. After deconvolution, the measured value of the HRMS of the heavy chain was 51551.58 Da.

### Preparation Example 34: Preparation of Site-Specific Glycoconjugated ADC Ab-G10-MMAE

Ab-G10-MMAE was prepared from compound N₃-PEG₄-VC-PAB-MMAE and non-natural sugar-engineered antibody Ab-G10 according to General Procedure III. After deconvolution, the measured value of the HRMS of the heavy chain was 51478.22 Da.

### Preparation Example 35: Preparation of Site-Specific Glycoconjugated ADC Ab-G13-DXd

Ab-G13-DXd was prepared from compound DBCO-GGFG-Dxd and non-natural sugar-engineered antibody Ab-G13 according to General Procedure II. After deconvolution, the measured value of the HRMS of the heavy chain was 50978.76 Da.

### Preparation Example 36: Preparation of Site-Specific Glycoconjugated ADC Ab-G20-DXd

Ab-G20-DXd was prepared from compound DBCO-GGFG-Dxd and non-natural sugar-engineered antibody Ab-G20 according to General Procedure II. After deconvolution, the measured value of the HRMS of the heavy chain was 50963.63 Da.

### Preparation Example 37: Preparation of Site-Specific Glycoconjugated ADC Ab-G21-DXd

Ab-G21-DXd was prepared from compound DBCO-GGFG-Dxd and non-natural sugar-engineered antibody Ab-G21 according to General Procedure II. After deconvolution, the measured value of the HRMS of the heavy chain was 50963.46 Da.

### Preparation Example 38: Preparation of Site-Specific Glycoconjugated ADC Ab-G22-DXd

Ab-G22-DXd was prepared from compound DBCO-GGFG-Dxd and non-natural sugar-engineered antibody Ab-G22 according to General Procedure II. After deconvolution, the measured value of the HRMS of the heavy chain was 52117.98 Da.

### Preparation Example 39: Preparation of Site-Specific Glycoconjugated ADC Ab-G23-DXd

Ab-G23-DXd was prepared from compound DBCO-GGFG-Dxd and non-natural sugar-engineered antibody Ab-G23 according to General Procedure II. After deconvolution, the measured value of the HRMS of the heavy chain was 52116.81 Da.

### Preparation Example 40: Preparation of Site-Specific Glycoconjugated ADC Ab-G24-DXd

Ab-G24-DXd was prepared from compound DBCO-GGFG-Dxd and non-natural sugar-engineered antibody Ab-G24 according to General Procedure II. After deconvolution, the measured value of the HRMS of the heavy chain was 52320.37 Da.

### Preparation Example 41: Preparation of Site-Specific Glycoconjugated ADC Ab-G25-DXd

Ab-G25-DXd was prepared from compound DBCO-GGFG-Dxd and non-natural sugar-engineered antibody Ab-G25 according to General Procedure II. After deconvolution, the measured value of the HRMS of the heavy chain was 52174.43 Da.

### Preparation Example 42: Preparation of Site-Specific Glycoconjugated ADC Ab-G27-DXd

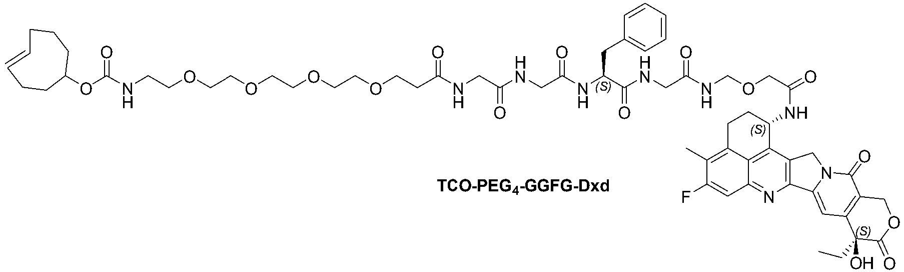

Ab-G27-DXd was prepared from compound TCO-PEG₄-GGFG-Dxd and non-natural sugar-engineered antibody Ab-G27 according to General Procedure IV. After deconvolution, the measured value of the HRMS of the heavy chain was 51234.41 Da.

### Preparation Example 43: Preparation of Site-Specific Glycoconjugated ADC Ab-G28b-DXd

Ab-G28b-DXd was prepared from compound DBCO-GGFG-Dxd and non-natural sugar-engineered antibody Ab-G28b according to General Procedure II. After deconvolution, the measured value of the HRMS of the heavy chain was 52432.16 Da.

### Preparation Example 44: Preparation of Site-Specific Glycoconjugated ADC Ab-G32-DXd

Ab-G32-DXd was prepared from compound DBCO-GGFG-Dxd and non-natural sugar-engineered antibody Ab-G32 according to General Procedure II. After deconvolution, the measured value of the HRMS of the heavy chain was 52101.64 Da.

### Preparation Example 45: Preparation of Site-Specific Glycoconjugated ADC Ab-G33-DXd

Ab-G33-DXd was prepared from compound DBCO-GGFG-Dxd and non-natural sugar-engineered antibody Ab-G33 according to General Procedure II. After deconvolution, the measured value of the HRMS of the heavy chain was 52304.05 Da.

### Preparation Example 46: Preparation of Site-Specific Glycoconjugated ADC Ab-G35-DXd

Ab-G35-DXd was prepared from compound DBCO-GGFG-Dxd and non-natural sugar-engineered antibody Ab-G35 according to General Procedure II. After deconvolution, the measured value of the HRMS of the heavy chain was 53271.19 Da.

### Preparation Example 47: Preparation of Site-Specific Glycoconjugated ADC Ab-G36-DXd

Ab-G36-DXd was prepared from compound DBCO-GGFG-Dxd and non-natural sugar-engineered antibody Ab-G36 according to General Procedure II. After deconvolution, the measured value of the HRMS of the heavy chain was 51018.64 Da.

### Preparation Example 48: Preparation of Site-Specific Glycoconjugated ADC Ab-G37-DXd

Ab-G37-DXd was prepared from compound DBCO-GGFG-Dxd and non-natural sugar-engineered antibody Ab-G37 according to General Procedure II. After deconvolution, the measured value of the HRMS of the heavy chain was 51032.43 Da.

### Preparation Example 49: Preparation of Site-Specific Glycoconjugated ADC Ab-G38-DXd-MMAE

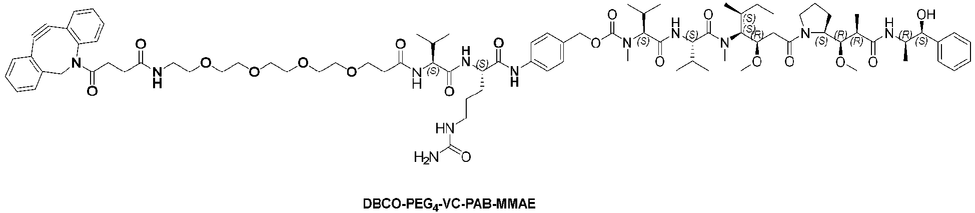

Ab-G38-DXd-MMAE was prepared from compounds TCO-PEG₄-GGFG-Dxd, DBCO-PEG₄-VC-PAB-MMAE, and non-natural sugar-engineered antibody Ab-G38 according to General Procedure VI. After deconvolution, the measured value of the HRMS of the heavy chain was 52887.29 Da.

### Preparation Example 50: Preparation of Site-Specific Glycoconjugated ADC Ab-G39-DXd-MMAE

Ab-G39-DXd-MMAE was prepared from compounds TCO-PEG₄-GGFG-Dxd, DBCO-PEG₄-VC-PAB-MMAE, and non-natural sugar-engineered antibody Ab-G39 according to General Procedure VI. After deconvolution, the measured value of the HRMS of the heavy chain was 52900.70 Da.

### Preparation Example 51: Preparation of Site-Specific Glycoconjugated ADC Ab-G40-DXd

Ab-G40-DXd was prepared from compound DBCO-GGFG-Dxd and non-natural sugar-engineered antibody Ab-G40 according to General Procedure II. After deconvolution, the measured value of the HRMS of the heavy chain was 50961.38 Da.

### Preparation Example 52: Preparation of Site-Specific Glycoconjugated ADC Ab-G42-DXd-MMAE

Ab-G42-DXd-MMAE was prepared from compounds TCO-PEG₄-GGFG-Dxd, DBCO-PEG₄-VC-PAB-MMAE, and non-natural sugar-engineered antibody Ab-G42 according to General Procedure VI. After deconvolution, the measured value of the HRMS of the heavy chain was 52914.89 Da.

### Preparation Example 53: Preparation of Site-Specific Glycoconjugated ADC Ab-G44-DXd-MMAE

Ab-G44-DXd-MMAE was prepared from compounds TCO-PEG₄-GGFG-Dxd, DBCO-PEG₄-VC-PAB-MMAE, and non-natural sugar-engineered antibody Ab-G44 according to General Procedure VI. After deconvolution, the measured value of the HRMS of the heavy chain was 53492.60 Da.

### Preparation Example 54: Preparation of Site-Specific Glycoconjugated ADC Ab-G45-DXd-MMAE

Ab-G45-DXd-MMAE was prepared from compounds TCO-PEG₄-GGFG-Dxd, DBCO-PEG₄-VC-PAB-MMAE, and non-natural sugar-engineered antibody Ab-G45 according to General Procedure VI. After deconvolution, the measured value of the HRMS of the heavy chain was 53300.04 Da.

### Preparation Example 55: Preparation of Site-Specific Glycoconjugated ADC Ab-G46-DXd

Ab-G46-DXd was prepared from compound DBCO-GGFG-Dxd and non-natural sugar-engineered antibody Ab-G46 according to General Procedure II. After deconvolution, the measured value of the HRMS of the heavy chain was 52174.17 Da.

### Preparation Example 56: Preparation of Site-Specific Glycoconjugated ADC Ab-G47-DXd

Ab-G47-DXd was prepared from compound DBCO-GGFG-Dxd and non-natural sugar-engineered antibody Ab-G47 according to General Procedure II. After deconvolution, the measured value of the HRMS of the heavy chain was 52173.90 Da.

### Preparation Example 57: Preparation of Site-Specific Glycoconjugated ADC Ab-G40-DBCO-PEG₄-VC-PAB-MMAE

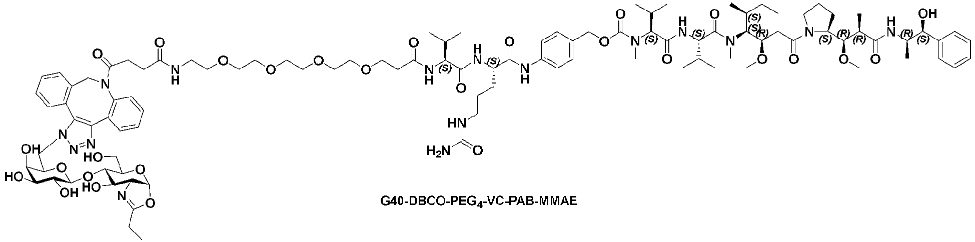

Ab-G40-DBCO-PEG₄-VC-PAB-MMAE was prepared from the wild-type antibody Pertuzumab and disaccharide conjugate G40-DBCO-PEG₄-VC-PAB-MMAE according to General Procedure V. After deconvolution, the measured value of the HRMS of the heavy chain was 51491.63 Da.

### Example 6 Preparation of Site-Specific Glycoconjugated ADCs Based on Non-Core Fucosylated Antibodies

The wild-type Pertuzumab was dissolved in a buffer consisting of 20 mM phosphate, 150 mM NaCl, and pH 7.4 to achieve a final mAb concentration of 10 mg/mL, hydrolyzed by adding wild-type Alfc (from *Lacticaseibacillus paracasei*, GENBANK accession number WP_012492118.1) at a final concentration of 0.5 mg/mL, incubated overnight at 37°C, and purified using Protein A magnetic beads to yield a defucosylated antibody (Ab-defuc) containing one N-acetylglucosamine.

The resulting defucosylated antibody, disaccharide linkers, and wild-type endoglycosidase Endo Se2 or Endo Si were placed in the same reaction system to achieve concentrations of 10 mg/mL, 1.67 mM (25-fold equivalents of the antibody concentration), and 0.4 mg/mL, respectively. The pH of the reaction system was adjusted to 7.4, and the reaction temperature was set to 25°C. After 6 h, 4 µL of sample was taken, and the reaction was stopped by adding 36 µL of PBS containing 0.1% formic acid and analyzed by LC-MS. The HRMS deconvoluted measured value of the heavy chain is shown in Table 3.

The defucosylated non-natural sugar-engineered antibodies Ab-defuc-G7, G20, G21, G23, G33, G35, G40 and the compound DBCO-GGFG-Dxd were used to obtain the corresponding ADCs according to General Procedure II, respectively. The HRMS deconvoluted measured value of the heavy chain is shown in Table 3.

The defucosylated non-natural sugar-engineered antibodies Ab-defuc-G38, G39, G42, G44, G45, and the compounds TCO-PEG₄-GGFG-Dxd and DBCO-PEG₄-VC-PAB-MMAE were used to obtain the corresponding ADCs according to General Procedure VI, respectively. The HRMS deconvoluted measured value of the heavy chain is shown in Table 3.

**Table 3 Preparation of site-specific glycoconjugated ADCs based on non-core fucosylated antibody**

| Antibody | Disaccharide linker | Transglycosylated antibody molecular weight (Da) | Linker-payload | Site-specific glycoconjugated ADC molecular weight (Da) |
|---|---|---|---|---|
| Ab-defuc | - | 49283.84 | - | - |
| Ab-defuc | G7 | 49664.23 | DBCO-GGFG-Dxd | 50818.78 |
| Ab-defuc | G20 | 49690.03 | DBCO-GGFG-Dxd | 50817.53 |
| Ab-defuc | G21 | 49689.14 | DBCO-GGFG-Dxd | 50817.49 |
| Ab-defuc | G23 | 49663.11 | DBCO-GGFG-Dxd | 51969.36 |
| Ab-defuc | G33 | 49901.12 | DBCO-GGFG-Dxd | 52157.45 |
| Ab-defuc | G35 | 49646.46 | DBCO-GGFG-Dxd | 53124.32 |
| Ab-defuc | G38 | 49872.52 | TCO-PEG₄-GGFG-Dxd/ DBCO-PEG₄-VC-PAB-MMAE | 52740.79 |
| Ab-defuc | G39 | 49885.94 | TCO-PEG₄-GGFG-Dxd/ DBCO-PEG₄-VC-PAB-MMAE | 52754.62 |
| Ab-defuc | G40 | 49687.05 | DBCO-GGFG-Dxd | 50815.49 |
| Ab-defuc | G42 | 49900.29 | TCO-PEG₄-GGFG-Dxd/ DBCO-PEG₄-VC-PAB-MMAE | 52768.25 |
| Ab-defuc | G44 | 50477.69 | TCO-PEG₄-GGFG-Dxd/ DBCO-PEG₄-VC-PAB-MMAE | 53346.16 |
| Ab-defuc | G45 | 50258.23 | TCO-PEG₄-GGFG-Dxd/ DBCO-PEG₄-VC-PAB-MMAE | 53152.98 |

As can be seen in Table 3, the ADC preparation methods provided by the present disclosure can be used for preparing non-core fucosylated ADCs.

### Example 7 Efficacy of Site-Specific Glycoconjugated Antibody-Drug Conjugates

### 1. Preparation of HER2-targeted site-specific glycoconjugated ADCs

### (1) Synthesis of Tmab-G7/G9/G22/G23/G24/G28b

The wild-type antibody Trastuzumab (Tmab), disaccharide oxazoline (i.e., compounds G7, G9, G22, G23, G24, G28b), wild-type endoglycosidase Endo Si (for compounds G7, G9, G23, and G28b) or Endo Se2 (for compounds G22 and G24) were placed in the same reaction system to achieve concentrations of 10 mg/mL, 1.67 mM (25-fold equivalents of antibody concentration), 0.6 mg/mL, respectively. The pH of the reaction system was adjusted to 7.4, and the reaction temperature was set to 25°C, with 800 rpm for 3 h. The small molecule compound and glycosidases were removed by purification using protein A to obtain the corresponding transglycosylated antibodies.

### (2) Preparation of corresponding ADCs

The prepared transglycosylated antibodies Tmab-G7, and DBCO-PEG₄-VC-PAB-MMAE were placed in the same reaction system to achieve concentrations of 5 mg/mL and 0.33 mM, respectively. The pH of the reaction system was adjusted to 7.4, and the mixture was incubated at 25°C overnight. The desired site-specific glycoconjugated ADC (i.e., Tmab-G7-MMAE) was obtained by ultrafiltration and analyzed by LC-MS, HIC, and SEC.

The prepared transglycosylated antibodies Tmab-G9, and N₃-PEG₄-VC-PAB-MMAE were placed in the same reaction system to achieve concentrations of 5 mg/mL and 0.33 mM, respectively. The pH of the reaction system was adjusted to 7.4, and the mixture was incubated at 25°C overnight. The desired site-specific glycoconjugated ADC (i.e., Tmab-G9-MMAE) was obtained by ultrafiltration and analyzed by LC-MS, HIC, and SEC.

The prepared transglycosylated antibodies Tmab-G22, G23, G24, G28b, and DBCO-PEG₄-VC-PAB-MMAE were placed in the same reaction system to achieve concentrations of 5 mg/mL and 0.33 mM, respectively. The pH of the reaction system was adjusted to 7.4, and after incubation at 25°C overnight, 8 equivalents of DBCO-PEG₄-VC-PAB-MMAE were added, and the total reaction time was 24 h. The desired site-specific glycoconjugated ADC (i.e., Tmab-G22/G23/G24/G28b-MMAE) was obtained by ultrafiltration and analyzed by LC-MS, HIC, and SEC.

ADC purity was determined by SEC for conjugated drugs and DAR values were determined by hydrophobic chromatography HIC-UPLC. Sample treatment: sample concentration of 1.0-5 mg/ml, filtered through a 0.22 µm filter. The general detection method includes:

### (1) Molecular exclusion chromatography SEC-HPLC

Sample treatment: sample concentration of 1.0-5 mg/ml, filtered through a 0.22µm filter; Column: TOSOH, TSKgel G3000SWxL, 5 µm, 7.8 mm×300 mm; Mobile phase: 0.2 M PB, 5-15% isopropanol, pH 7.0, flow rate: 0.5-1 mL/min; Detection wavelength: 280 nm & 248 nm; Column temperature: room temperature; Loading amount: 30 µg; SEC chromatography elution method: isocratic elution.

### (2) Hydrophobic interaction chromatography HIC-UPLC

Column: TOSOH, HIC TSK Butyl-NPR, 2.5 µm, 4.6 mm×100 mm; Column temperature: room temperature; Mobile phase A: 0.05 M PB, 1.2 M ammonium sulfate, pH 7.0; Mobile phase B: 0.05 M PB, pH 7.0, 20% isopropanol; Flow rate: 0.5 mL/min; Loading amount: 30 µg; Gradient method: increase from 0% to 100% over 20 minutes; Detection wavelength: 280 nm & 248 nm.

Wherein, PB refers to sodium phosphate buffer mainly composed of disodium hydrogen phosphate and sodium dihydrogen phosphate. Phosphate buffer solutions of different pH values with disodium hydrogen phosphate-sodium dihydrogen phosphate are usually prepared using the same concentration of sodium dihydrogen phosphate and disodium hydrogen phosphate solutions.

The ADC products obtained by coupling in this manner were tested for HIC DAR values and SEC purity obtained for HIC-UPLC and SEC-HPLC, respectively.

The quality control data for the HER2-targeted site-specific glycoconjugated ADCs prepared above are shown in Table 4.

**Table 4 The quality control data for the HER2-targeted site-specific glycoconjugated ADCs**

| ADC name | Target | Payload | Molecular weight (Da) | Drug antibody ratio (HIC) | SEC (%) |
|---|---|---|---|---|---|
| Tmab-G7-MMAE | HER2 | MMAE | 149926.45 | 1.95 | 99.29 |
| Tmab-G9-MMAE | HER2 | MMAE | 150041.06 | 2 | 98.21 |
| Tmab-G22-MMAE | HER2 | MMAE | 153293.84 | 3.64 | 99.61 |
| Tmab-G23-MMAE | HER2 | MMAE | 153293.86 | 3.84 | 99.60 |
| Tmab-G24-MMAE | HER2 | MMAE | 153699.92 | 3.71 | 99.86 |
| Tmab-G28b-MMAE | HER2 | MMAE | 153922.45 | 3.7 | 99.77 |

### 2. In vitro efficacy of the HER2-targeted site-specific glycoconjugated ADCs

The purpose of this experiment is to test the *in vitro* inhibitory activity of the ADC compounds of the present disclosure against SK-BR-3 (human breast adenocarcinoma cells), NCI-N87 (human gastric cancer cells), and MDA-MB-468 (human breast cancer cells).

Tumor cells SK-BR-3 (source:: Cell Resource Center, Shanghai Institute of Life Sciences, Chinese Academy of Sciences), NCI-N87 (source: Cell Resource Center, Shanghai Institute of Life Sciences, Chinese Academy of Sciences), or MDA-MB-468 (source: National Collection of Authenticated Cell Cultures, Chinese Academy of Sciences) in the logarithmic growth phase were added to the cell plates at a quantity of 2000 cells/well, and the cell plates were incubated in a 5% CO₂ cell incubator at 37°C for 12-16 hours. 100 µL of samples (starting at 50 µg/mL, with a 5-fold dilution and 9 concentrations) were added to each well, gently shaken, and then incubated in an incubator. After incubation for 144 hours, 70 µL of CellTiter-Glo^{™} (Promega, Catalog Number: G7572) working solution was added, and the cells were lysed by gentle shaking. The plates were read on a microplate reader. The cell proliferation inhibition rate was calculated using the formula: Cell proliferation inhibition rate = (1-sample well/control well) ×100%. Using GraphPad Prism 8.0 software, the data were plotted with the logarithm of sample concentration on the x-axis and cytotoxicity (%) on the y-axis, and analyzed by nonlinear regression (curve fit) to obtain the IC₅₀ value of each test article. The detailed results are shown in Table 5.

**Table 5 In vitro antitumor activity assay of the ADC compounds of the present disclosure**

| ADC compound | SK-BR-3 (HER2+++) | | NCI-N87 (HER2+++) | | MDA-MB-468 (HER2-) | |
|---|---|---|---|---|---|---|
| | IC₅₀ (µg/mL) | Max inhibition (%) | IC₅₀ (µg/mL) | Max inhibition (%) | IC₅₀ (µg/mL) | Max inhibition (%) |
| Tmab-G7-MMAE | 0.004 | 92 | 0.005 | 81 | 44 | 60 |
| Tmab-G9-MMAE | 0.003 | 92 | 0.003 | 80 | 34 | 76 |
| Tmab-G22-MMAE | 0.002 | 92 | 0.003 | 82 | 35 | 77 |
| Tmab-G23-MMAE | 0.001 | 92 | 0.002 | 84 | 31 | 81 |
| Tmab-G24-MMAE | 0.001 | 92 | 0.002 | 82 | 19 | 84 |
| Tmab-G28b-MMAE | 0.002 | 92 | 0.003 | 81 | 15 | 86 |

As can be seen in Table 5, the ADC drugs in the present disclosure have significant proliferation inhibitory activity against HER2 positive cells SK-BR-3 and NCI-N87, but show weak proliferation inhibitory activity against HER2 negative cells MDA-MB-468, thereby exhibiting good selectivity.

### 3. In vivo efficacy of HER2-targeted site-specific glycoconjugated ADCs

BALB/c Nude mice (purchased from Beijing Vital River) were used as test animals to evaluate the efficacy of anti-HER2 ADCs administered by tail vein injection to human gastric cancer cell NCI-N87 xenografts in nude mice.

Mice were subcutaneously inoculated into the right axillary fossa with NCI-N87 cells (source: ATCC) (5×10⁶/mouse, with 50% matrix gel), and tumors were allowed to grow for 7 days until the average tumor volume reached about 150 mm³. The animals were then randomly grouped based on the tumor volume (D7), with 7 mice per group.

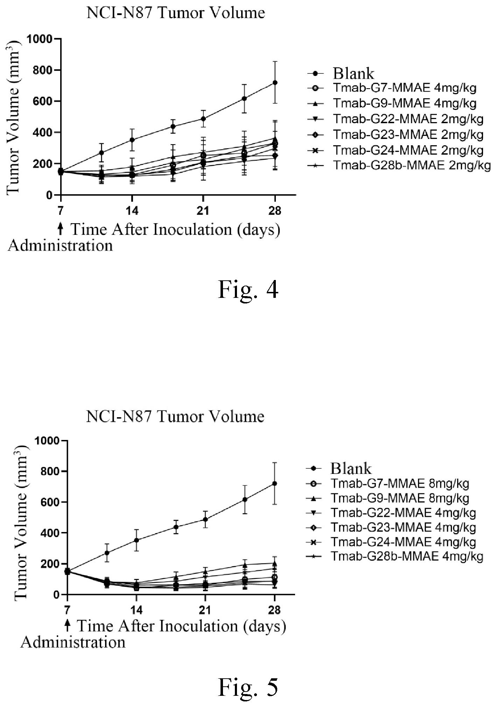

A single dose was administered via tail vein injection, and tumor volume and body weight were measured twice a week, with data recorded. The tumor growth inhibition (TGI) rate (%) = [1-(T₂₈-T₇)/ (V₂₈-V₇)] ×100, where T₂₈ and T₇ represent the tumor volumes of the experimental groups on day 28 and day 7 after inoculation, respectively, and V₂₈ and V₇ represent the tumor volumes of the blank control group (Vehicle, PBS) on day 28 and day 7 after inoculation, respectively. The detailed experimental results at the end of the study on day 28 after inoculation are shown in Table 6 and Figures 4-5.

**Table 6 Evaluation of in vivo efficacy of ADC compounds of the present disclosure**

| ADC compound | Dosing | Tumor growth inhibition rate |
|---|---|---|
| Tmab-G7-MMAE | 4 mpk | 68.6% |
| Tmab-G9-MMAE | 4 mpk | 62.4% |
| Tmab-G22-MMAE | 2 mpk | 85.3% |
| Tmab-G23-MMAE | 2 mpk | 82.2% |
| Tmab-G24-MMAE | 2 mpk | 74.1% |
| Tmab-G28b-MMAE | 2 mpk | 68.0% |
| Tmab-G7-MMAE | 8 mpk | 106.5% |
| Tmab-G9-MMAE | 8 mpk | 90.6% |
| Tmab-G22-MMAE | 4 mpk | 115.6% |
| Tmab-G23-MMAE | 4 mpk | 111.5% |
| Tmab-G24-MMAE | 4 mpk | 110.8% |
| Tmab-G28b-MMAE | 4 mpk | 96.4% |

As can be seen in Table 6, the ADC drugs constructed using the disaccharide linker in the present disclosure exhibit excellent antitumor activity against HER2-overexpressing NCI-N87 xenografts, suggesting that the enzyme of the present disclosure is suitable for industrial application in the preparation of sugar chain-remodeled ADCs.

The embodiments of the present disclosure described above are exemplary only, and those skilled in the art will recognize or be able to identify countless equivalents of specific compounds, materials, and methods without the need for experiments beyond conventional testing. All such equivalents are within the scope of this disclosure and are included in the claims.

## Claims

1. An enzyme having the amino acid sequence set forth in SEQ ID NO: 1, wherein the enzyme exhibits sugar chain hydrolysis and/or sugar chain transfer activity.

2. A method for sugar chain remodeling of a polypeptide or protein, comprising the following steps:
a) introducing Endo Se2 or Endo Si;
b) introducing a polypeptide or protein comprising at least one N-acetylglucosamine or core fucosylated N-acetylglucosamine;
c) providing a disaccharide linker or disaccharide conjugate; and
d) using Endo Se2 or Endo Si to transfer the disaccharide linker or disaccharide conjugate to the polypeptide or protein in step (b), to provide a novel sugar chain-modified polypeptide or protein;
wherein the sequence of Endo Se2 is set forth in SEQ ID NO: 1, and the sequence of Endo Si is set forth in SEQ ID NO: 2.

3. The method according to claim 2, wherein the polypeptide or protein is an antibody or a protein comprising the Fc region of the antibody.

4. The method according to claim 2, wherein the polypeptide or protein comprising at least one N-acetylglucosamine or core fucosylated N-acetylglucosamine is obtained by hydrolysis of an N-glycan-containing polypeptide or protein using an endoglycosidase, by recombinant expression, or by chemical synthesis.

5. The method according to claim 4, wherein the N-glycan is a natural or non-natural complex, high-mannose, hybrid N-glycan.

6. The method according to claim 4, wherein the endoglycosidases include Endo Se2, Endo Si, Endo S, Endo S2, Endo F3, and mutants thereof.

7. The method according to claim 4, wherein the N-glycan-containing polypeptide or protein achieves sugar chain remodeling in one step by introducing Endo Se2 or Endo Si.

8. The method according to claim 4, wherein the N-glycan-containing polypeptide or protein achieves sugar chain remodeling in one step by simultaneously introducing Endo Se2 or Endo Si with another endoglycosidase or multiple endoglycosidases.

9. The method according to claim 2, wherein the disaccharide linker or disaccharide conjugate comprises at least one oxazolinylated or thiazolinylated monosaccharide, or monosaccharide modified/engineered with a bioorthogonal functional group.

10. The method according to claim 9, the disaccharide linker or disaccharide conjugate is selected from the following structures:

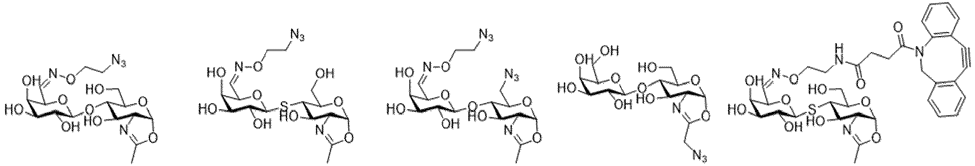

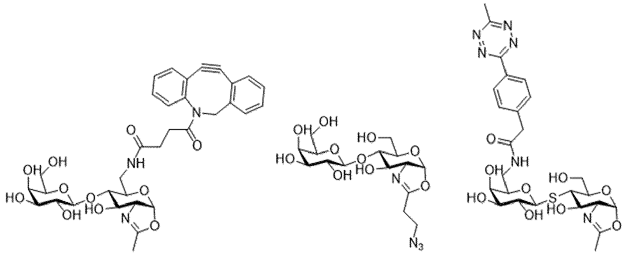

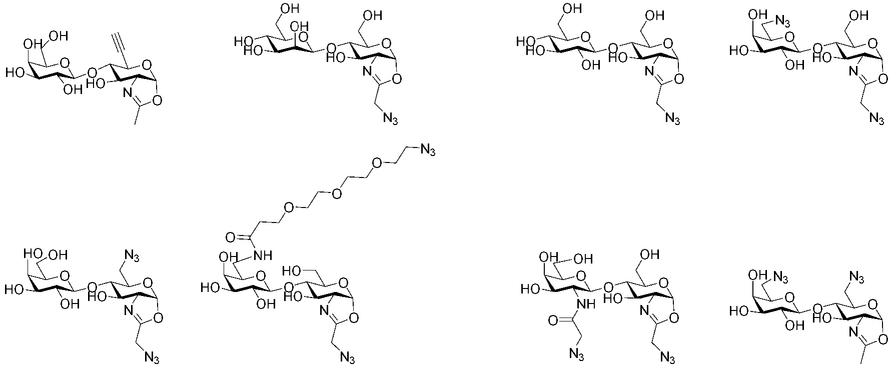

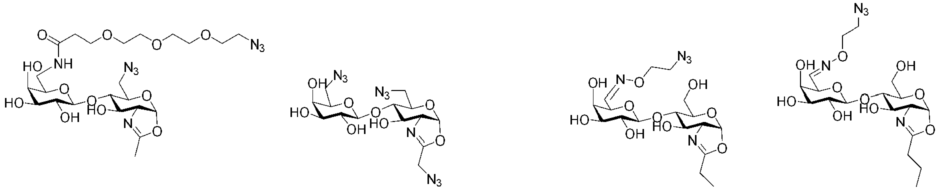

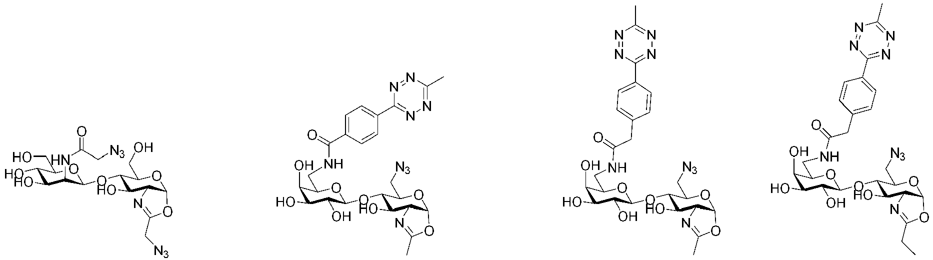

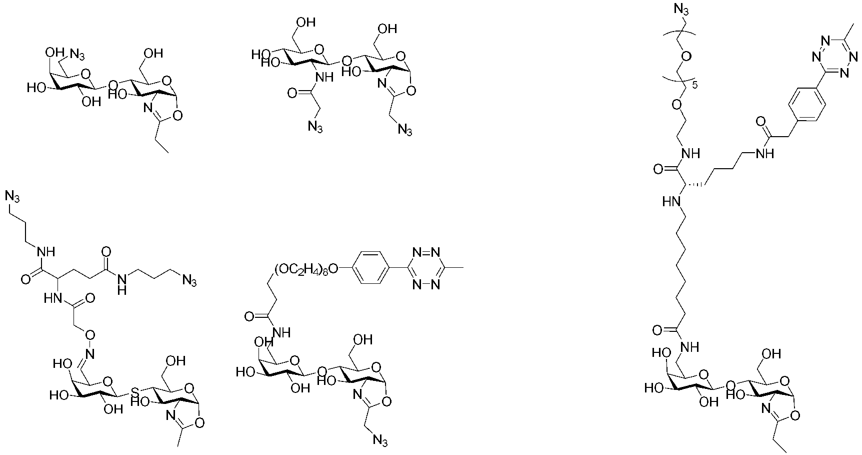

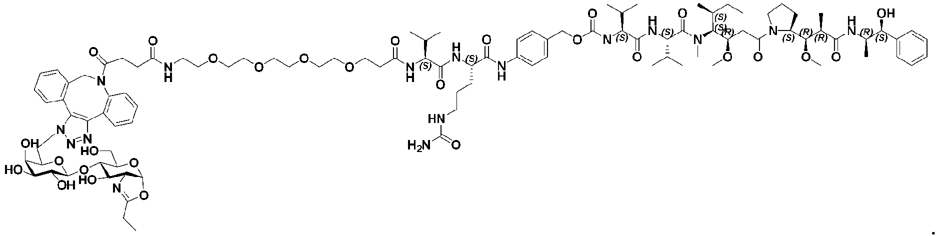

11. Use of Endo Se2 set forth in SEQ ID NO: 1 or Endo Si set forth in SEQ ID NO: 2 for transferring a disaccharide linker or disaccharide conjugate in glycosyl modification.
